Europäisches Patentamt

(19) European Patent Office

Office européen des brevets

(11) Publication number: **0 107 949**
**B1**

(12) **EUROPEAN PATENT SPECIFICATION**

(45) Date of publication of patent specification: **13.01.88**

(21) Application number: **83306290.4**

(22) Date of filing: **17.10.83**

(51) Int. Cl.⁴: **A 61 F 7/10,** A 61 F 7/00, A 61 N 5/02

(54) **A cooler for human tissue for use during hyperthermia treatment against cancer.**

(30) Priority: **25.10.82 JP 162214/82**

(43) Date of publication of application:
**09.05.84 Bulletin 84/19**

(45) Publication of the grant of the patent:
**13.01.88 Bulletin 88/02**

(84) Designated Contracting States:
**AT BE DE FR IT NL SE**

(56) References cited:
**WO-A-80/01462**
**DE-A-2 052 778**
**DE-A-2 648 908**
**DE-C- 173 788**
**GB-A-1 355 373**
**GB-A-1 448 068**
**GB-A-1 448 068**
**US-A- 267 435**
**US-A-1 902 016**
**US-A-1 991 784**
**US-A-2 397 232**
**US-A-3 307 553**
**US-A-3 859 986**

(73) Proprietor: **JUNKOSHA CO. LTD.**
**25-25, Miyasaka 2-chome**
**Setagaya-ku Tokyo156 (JP)**

(72) Inventor: **Suzuki, Hirosuke**
**21-8 Kotesashi-Machi 4-chome Tokorocawa-Shi**
**Saitama (JP)**
Inventor: **Kobayashi, Satoru**
**927-3 Aka-Nakacawa Ohaza-Takahagi Hitaka-Machi**
**Iruma-Gun Saitama (JP)**

(74) Representative: **Taylor, Derek George et al**
**MATHISEN, MACARA & CO. The Coach House**
**6-8 Swakeleys Road**
**Ickenham Uxbridge UB10 8BZ (GB)**

(56) References cited:
**US-A-4 082 893**
**US-A-4 149 541**
**US-A-4 364 985**
**US-A-4 397 314**

Courier Press, Leamington Spa, England.

## Description

The present invention relates to apparatus for use in the heat treatment of cancerous tumours. In such treatment, called hyperthermic treatment, a cancerous region of a human is heated, in particular to 41-43°C, with heat producing microwaves to destroy the cancerous cells.

In conventional heat therapy apparatus of this kind a heater, in the form of a microwave antenna, is disposed on human epithelial tissue to heat the cancerous region which may located deep in the human body.

Unfortunately, in such therapy, the density of electromagnetic waves is highest just below the antenna and the temperature in the cancerous region should be kept constant. Therefore, the epithelial tissue may be higher in temperature than the cancerous region, and cooling of the epithelial tissue is required.

It is known from WO—A—80/01462 and US—A—4,140,130 to provide apparatus for use in the treatment of cancerous tumours which comprises a microwave heater, the apparatus incorporating a tube through which refrigerant can be passed. The preamble of claims 1, 6 are based on the respective disclosures of these documents. It is also known from GB—A—1355373 to make a porous tube of polytetrafluoroethylene.

According to the present invention there is provided apparatus for use in the heat treatment of cancerous tumours comprising a microwave heater and a cooling device for cooling epithelial tissue, said cooling device comprising at least one tube, disposed between the microwave heater and the cancerous region to be treated and through which a refrigerant can be caused to flow, characterised in that the tube is made of a porous, expanded polytetrafluorethylene resin having interconnecting pores.

Preferably, the tube is wound into the form of a flat coil at least one side of the tube being held in place by a sheet of porous, expanded polytetrafluoroethylene having interconnecting pores.

The present invention also provides a cooling device for use in cooling the tissue surrounding a cancerous tumour in a patient undergoing heat treatment of the cancerous tumour by microwave energy, the device comprising at least one tube through which a refrigerant liquid can be caused to flow, characterized in that the tube is disposed in a predetermined convoluted configuration and made of a porous, expanded polytetrafluoroethylene resin having interconnecting pores.

Other preferred embodiments of the invention are set-out in dependent claims.

The invention will now be particularly described with reference to the accompanying drawings in which:—

Fig. 1 is a diagrammatic view of heat treatment apparatus according to the present invention;

Fig. 2 is a perspective view of a cooling device according to the invention;

Fig. 3 is a fragmentary plan view of another cooling device according to the invention;

Fig. 4 is a fragmental side elevation of the tube used in the cooling device of figure 3 showing inlet/outlet ports.

Fig. 5 is a perspective view of still another cooling device according to the invention;

Fig. 6 is a cross-sectional view of the device shown in Fig. 5 taken along the line VI—VI thereof;

Fig. 7 is a top plan view of a further embodiment of the invention; and

Fig. 8 is a cross-sectional view taken along line VIII—VIII of Fig. 7.

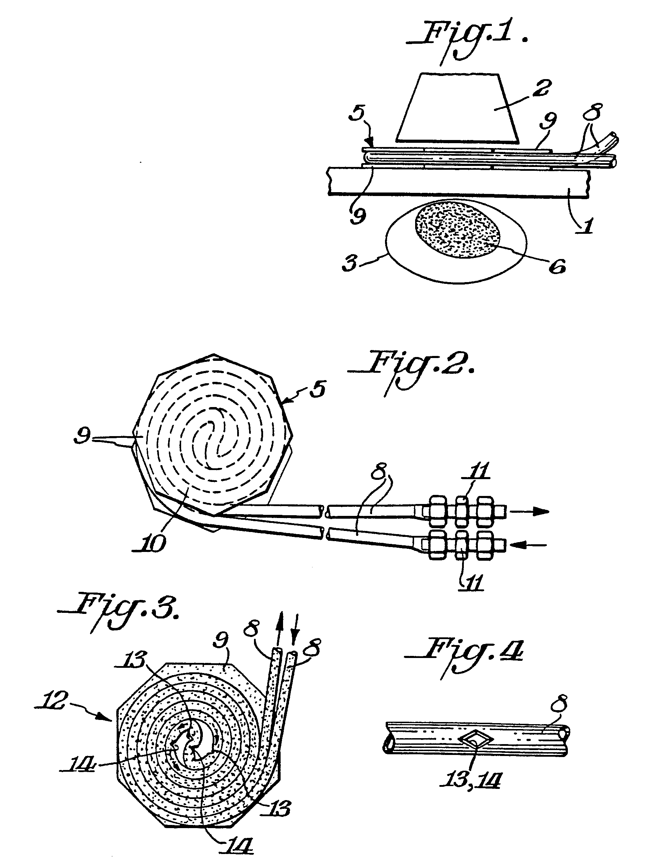

As illustrated in Figure 1, apparatus for the heat treatment of cancerous tumours can comprise a heater, such as a microwave antenna 2, which is disposed on epithelial tissue 1 to heat the cancerous region 6 of the cancerous organ 3 which may be located deep in the human body.

In accordance with one aspect of the present invention, the heat therapy apparatus includes a cooling device 5. The device 5 is disposed between the epithelial tissue 1 and the microwave antenna 2 which acts as a heater. The device 5 is placed in direct contact with the tissue 1 and includes a tube 8 through which a refrigerant is caused to flow, the tube consisting of porous polytetrafluoroethylene having interconnecting pores. Accordingly, the cooling device is flexible and conforms to the epithelial tissue 1, and hence the device can effectively cool the tissue. Further, since the device 5 is made of inert polytetrafluorothylene it does not cause discomfort or physical disorder to the human body tissue 1.

In addition, since the portion of the cooling device 5 through which refrigerant is circulated has a great number of interconnecting pores, even if the refrigerant is warmed by the heater, the refrigerant is vaporised thereby and a large quantity of heat is removed, thereby cooling the tissue more effectively. Accordingly, even if a cancerous region 6 of the cancerous organ 3, or the like, is hypertrophied, the required heat energy can be given to the cancerous region while cooling noncancerous regions effectively with the device 5. Consequently, the cancerous region 6 can be treated effectively. Moreover, since the portion through which refrigrant is passed is made of polytetrafluoroethylene it has a small dielectric loss. It follows that the tube is not heated by electromagnetic waves and that the heating efficiency is not lowered substantially.

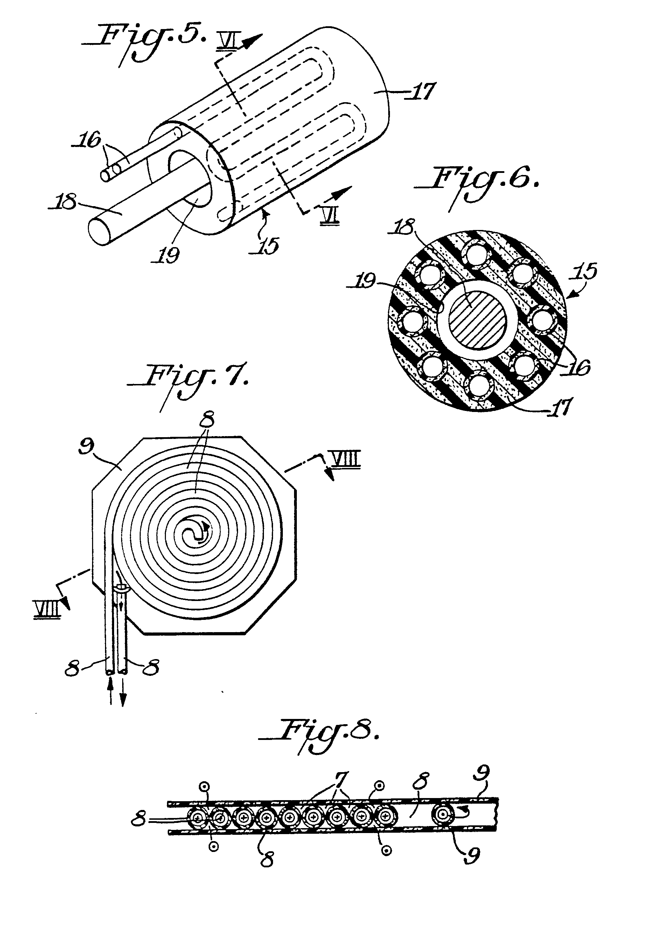

Referring next to Fig. 2, an example of the cooling device 5 according to the invention is shown. The device 5 comprises a tube 8 of porous, expanded polytetrafluoroethylene resin having interconnected pores. The tube 8 is folded in two and wound into the form of a coil around the folded portion. The two sides of the coil can be held in position by sheets 9 of a porous fluorocarbon resin having interconnecting pores, whereby a coiled portion 10 through which the refrigerant passes in contra-flow is formed. Both ends of the tube 8 are provided with couplings 11.

In the cooling device 5 of Fig. 2, the sheets 9, holding the coiled portion 10, have interconnecting pores. Therefore, if each of the sheets is in contact with the tube 8 at a point, the cooling due to the vaporization of refrigerant is not hindered. Although the coiled portion is held by the sheets 9 on both sides, in the above embodiment, it is also possible to mount one sheet 9 on only one side of the coil. The tube 8 and the sheets 9 can be fabricated by the process dislosed in Japanese Patent Publication No. 18991/1976, for example.

Fig. 3 is a fragmentary plan view of another cooling device according to the invention, the device being indicated by reference numeral 12. The cooling device is similar to the cooling device having the coiled portion 10 shown in Fig. 2 except in the following respects. The tube 8 and the sheets 9 are held in liquid-tight manner by leaving no space between the neighbouring turns, especially central turns, of the tube 8 made of porous, expanded polytetrafluoroethylene resin having interconnecting pores (contrary to the diagrammatic showing of Fig. 3). The coil is centrally provided with a refrigerant outlet port 13 and a refrigerant inlet port 14. Thus, in the cooling device 12 of this example, the cooling capability in the center is enhanced to some extent and can be preferably used together with a heater in which the heat energy density in the center is higher.

Other kinds of cooling devices can be fabricated using the same principles as in the cooling device 12 shown in Fig. 3. In particular, as shown in Figs. 7 and 8, a tube of porous polytetrafluoroethylene having interconnecting pores is first prepared. Then, the tube is wound into the form of a coil around one end of the tube. The top and bottom surfaces of the coil are joined and held in liquid-tight manner by sheets of a porous fluorocarbon resin having interconnecting pores. The tube is used as a refrigerant inlet tube. A passage 7 is formed between the outer boundaries of the tube and the inner surfaces of the sheets and has a substantially triangular cross section such that the refrigerant discharged from the inner end of the tube returns through this passage. In this case, it is required that the end of the return passage is provided with a refrigerant discharge tube as shown.

In the cooling device constructed as described just above, the return passage for the refrigerant has a triangular cross section and is provided between the neighbouring turns of the coil. Consequently, the velocity of the returning refrigerant is increased, and the area through which heat is dissapated is also enlarged. Further, the distance between the refrigerant and the object to be cooled is reduced, and the thermal resistance is decreased. In this manner, the cooling device can provide good cooling efficiency.

Fig. 5 is a perspective view of still another cooling device 15 according to the present invention. The device 15 is adapted to be inserted in a body cavity for treating a cancerous tumour formed in the cavity. The cooling device 15 comprises a tube 16 of porous, expanded polytetrafluoroethylene resin having interconnecting pores, the tube 16 being disposed overlapping on itself as shown. The tube is held in a cylindrical configuration by a body 17 of a porous fluoroethylene resin having interconnecting pores such that a heater, for example a microwave antenna 18, can be inserted in the center 19 of the cylinder.

In the embodiment described just above, the cooling device 15 fits the internal wall of a body cavity or the region surrounding the embedded cooling device, permitting favourable cooling of noncancerous regions. Further, because it consists of inert expanded polytetrafluoroethylene, it gives no feeling of uneasiness or physical disorder to the region of the human body in which the device is inserted or embedded. Quite advantageously, this permits a stable and continuous treatment.

Fig. 6 is a cross-sectional view of the device of Fig. 5 showing tube 16, cylindrical body 17 and microwave antenna 18 inserted into cavity 19.

As described thus far, in accordance with the present invention, a cooling device for heat therapy for cancer is provided to be disposed between a heater and a cancerous region to be treated, and which includes at least one tube through which a refrigerant liquid can be caused to flow, the tube being disposed in a predetermined convoluted configuration and consisting of porous, expanded polytetrafluoroethylene having interconnecting pores. Thus, it is possible to cool noncancerous regions efficiently without substantially reducing the heating efficiency of the heater, so that the required heat energy is provided to the cancerous region, thereby achieving improved treatment. Hence, the device can be installed on the surface of the body or in a body cavity, thus leading to efficient cooling. Another advantage is that it results in no physical disorder to the human body. Furthermore, because the portion through which the refrigerant is circulated has a number of interconnecting pores, it can provide a large cooling capability by virtue of the heat of vaporization of the refrigerant, inspite of the presence of the heater.

Suitable refrigerants include water and alcohol. Bonding between the porous tube and the porous sheets can be achieved by using suitable adhesives.

**Claims**

1. Apparatus for use in the heat treatment of cancerous tumours comprising a microwave heater (2) and a cooling device (5) for cooling epithelial tissue, said cooling device comprising at least one tube (8), disposed between the microwave heater and the cancerous region (6) to be treated and through which a refrigerant can be caused to flow, characterised in that the tube is made of a porous, expanded polytetrafluoroethylene resin having interconnecting pores.

2. Apparatus according to claim 1 characterised in that said tube (8) is wound into the form of a flat coil, at least one side of the tube being held in place by a sheet (9) of porous, expanded polytetrafluoroethylene having interconnecting pores.

3. Apparatus according to claim 2 characterised in that the coil comprises a coiled inlet portion and a coiled outlet portion disposed between the turns of the inlet portion.

4. Apparatus according to claim 2 or claim 3 characterised in that both sides of the tube (8) are held in place by sheets (9) of a porous, expanded polytetrafluoroethylene resin, and the peripheral portion of the coil of the tube is placed in a liquid-tight relation with the sheets, the tube being centrally provided with a refrigerant inlet port and a refrigerant outlet port.

5. Apparatus according to claim 1 characterised in that said tube (16) is disposed in a zig-zag fashion and held so as to form a cylinder, and wherein said tube is centrally provided with a portion into which the microwave heater (18) can be fitted.

6. A cooling device (5) for use in cooling the tissue surrounding a cancerous tumour (6) in a patient undergoing heat treatment of the cancerous tumour by microwave energy, the device comprising at least one tube (8) through which a refrigerant liquid can be caused to flow, characterised in that the tube (8) is disposed in a predetermined convoluted configuration and made of a porous, expanded polytetrafluoroethylene resin having interconnecting pores.

7. A cooling device according to claim 6 characterised in that the tube (8) is wound in a flat coil of closely adjacent turns.

8. A cooling device according to claim 7 characterised in that at least one side of the tube (8) is held in place by a sheet (9) of porous, expanded polytetrafluoroethylene having interconnecting pores.

9. A cooling device according to claim 7 characterised in that the coil comprises a coiled inlet portion and a coiled outlet portion disposed between the turns of the inlet portion.

10. A cooling device according to claim 7 characterised by a pair of sheets (9) of a porous expanded polytetrafluoroethylene resin having interconnecting pores, the sheets being joined to both sides of at least one coiled portion of the inlet tube in a liquid-tight manner to support said coiled portion.

## Patentansprüche

1. Vorrichtung zur Verwendung bei der Wärmebehandlung von krebsartigen Tumoren, mit einem Mikrowellenerhitzer (2) und einer Kühleinrichtung (5) zum Kühlen von Epithelgewebe, wobei die Kühleinrichtung wenigstens einen Schlauch (8) aufweist, der zwischen dem Mikrowellenerhitzer und dem zu behandelnden kanzerösen Bereich (6) angeordnet ist, und durch den ein Kühlmittel strömen gelassen werden kann, dadurch gekennzeichnet, daß der Schlauch aus porösem, geschäumten Polytetrafluoräthylenharz mit verbindenden Poren hergestellt ist.

2. Vorrichtung nach Anspruch 1, dadurch gekennzeichnet, daß der Schlauch (8) in Form einer flachen Rolle gewunden ist, wobei zumindest eine Seite des Schlauches durch eine Folie (9) aus porösem, geschäumten Polytetrafluoräthylen mit verbindenden Poren an ihrem Platz festgehalten ist.

3. Vorrichtung nach Anspruch 2, dadurch gekennzeichnet, daß die Rolle einen aufgewickelten Einlaßteil und einen aufgewickelten Auslaßteil zwischen den Windungen des Einlaßteiles aufweist.

4. Vorrichtung nach Anspruch 2 oder 3, dadurch gekennzeichnet, daß beide Seiten des Schlauches (8) durch Folien (9) aus einem porösen, geschäumten Polytetrafluoräthylen am Platz gehalten sind und der Umfangsrandteil der Schlauchrolle in flüssigkeitsdichter Beziehung zu den Folien angeordnet ist, wobei der Schlauch mittig mit einer Kühlmitteleinlaßöffnung und einer Kühlmittelauslaßöffnung versehen ist.

5. Vorrichtung nach Anspruch 1, dadurch gekennzeichnet, daß der Schlauch (16) zick-zackförmig angeordnet und so gehalten ist, daß er einen Zylinder bildet, und wobei der Schlauch mittig mit einem Teil versehen ist, in den der Mikrowellenerhitzer (18) eingesetzt werden kann.

6. Kühlvorrichtung (5) zur Verwendung beim Kühlen des Gewebes, das einen krebsartigen Tumor (6) eines Patienten umgibt, der eine Wärmebehandlung des krebsartigen Tumors durch Mikrowellenenergie erfährt, wobei die Einrichtung wenigstens einen Schlauch (8) aufweist, durch den eine Kühlflüssigkeit strömen gelassen werden kann, dadurch gekennzeichnet, daß der Schlauch (8) in einer vorherbestimmten gewundenen Gestalt angeordnet und aus einem porösen, geschäumten Polytetrafluoräthylenharz mit verbindenden Poren hergestellt ist.

7. Kühleinrichtung nach Anspruch 6, dadurch gekennzeichnet, daß der Schlauch zu einer flachen Rolle mit eng benachbarten Windungen gewunden ist.

8. Kühleinrichtung nach Anspruch 7, dadurch gekennzeichnet, daß zumindest eine Seite des Schlauches (8) durch eine Folie (9) aus porösem, geschäumten Polytetrafluoräthylen mit verbindenden Poren an ihrem Platz gehalten ist.

9. Kühleinrichtung nach Anspruch 7, dadurch gekennzeichnet, daß die Rolle einen aufgewickelten Einlaßteil und einen aufgewickelten Auslaßteil zwischen den Windungen des Einlaßteiles aufweist.

10. Kühleinrichtung nach Anspruch 7, gekennzeichnet durch ein Paar von Folien (9) aus einem porösen, geschäumten Polytetrafluoräthylenharz mit verbindenden Poren, wobei die Folien mit beiden Seiten zumindest eines aufgerollten Teiles des Einlaßschlauches flüssigkeitsdicht verbunden sind, um den aufgerollten Teil zu halten.

## Revendications

1. Appareil utilisable dans le traitement thermique de tumeurs cancéreuses, comprenant un appareil chauffant à micro-ondes (2) et un dispositif de refroidissement (5) pour le refroidissement du tissu épithélial, ce dispositif de refroidissement comportant au moins un tube (8) disposé entre le dispositif chauffant à micro-ondes et la zone cancéreuse (6) à traiter, et dans lequel un réfrigérant peut être amené à circuler, caractérisé en ce que ce tube est fait d'une résine de polytétrafluoréthylène expansée, poreuse, comportant des pores communiquant entre eux.

2. Appareil suivant la revendication 1, caractérisé en ce que le tube susdit (8) est enroulé sous la forme d'un serpentin plat, au moins un côté du tube étant maintenu en place par une feuille (9) de polytétrafluoréthylène expansé poreux, comportant des pores communiquant entre eux.

3. Appareil suivant la revendication 2, caractérisé en ce que le serpentin comprend une partie d'entrée spiralée et une partie de sortie spiralée disposée entre les spires de la partie d'entrée.

4. Appareil suivant la revendication 2 ou la revendication 3, caractérisé en ce que les deux côtés du tube (8) sont maintenus en place par des feuilles (9) d'une résine de polytétrafluoréthylène expansée, poreuse, et en ce que la partie périphérique du serpentin tubulaire se trouve dans un état étanche aux liquides en relation avec ces feuilles, le tube comportant centralement un orifice d'entrée de réfrigérant et un orifice de sortie de réfrigérant.

5. Appareil suivant la revendication 1, caractérisé en ce que le tube (16) est disposé en zig-zag et est maintenu de manière à former un cylindre, ce tube comportant centralement une partie dans laquelle peut être monté le dispositif chauffant à micro-ondes (18).

6. Dispositif de refroidissement (5) utilisable pour le refroidissement du tissu entourant une zone cancéreuse (6) sur un patient subissant un traitement thermique de la tumeur cancéreuse par une énergie à micro-ondes, ce dispositif comprenant au moins un tube (8) dans lequel un liquide réfrigérant peut être amené à circuler, caractérisé en ce que le tube (8) est disposé suivant une allure spiralée prédéterminée et est fait d'une résine de polytétrafluoréthylène expansée poreuse, comportant des pores communiquant entre eux.

7. Dispositif de refroidissement suivant la revendication 6, caractérisé en ce que le tube (8) est enroulé sous forme d'un serpentin plat dont les spires sont étroitement adjacentes.

8. Dispositif de refroidissement suivant la revendication 7, caractérisé en ce qu'au moins un côté du tube (8) est maintenu en place par une feuille (9) de polytétrafluoréthylène expansé poreux comportant des pores communiquant entre eux.

9. Dispositif de refroidissement suivant la revendication 7, caractérisé en ce que le serpentin comporte une partie d'entrée spiralée et une partie de sortie spiralée disposée entre les spires de la partie d'entrée.

10. Dispositif de refroidissement suivant la revendication 7, caractérisé en ce qu'il comporte une paire de feuilles (9) d'un résine de polytétrafluoréthylène expansée, poreuse, comportant des pores communiquant entre eux, ces feuilles étant jointes aux deux côtés d'au moins une partie spiralée du tube d'entrée, d'une manière étanche aux liquides, pour soutenir cette partie spiralée.

0 107 949

Fig.1.

Fig.2.

Fig.3.

Fig.4.

1

*Fig.5.*

*Fig.6.*

*Fig.7.*

*Fig.8.*